# EUROPEAN PATENT SPECIFICATION

(11) **EP 2 692 413 B1**
(45) Date of publication and mention of the grant of the patent: **25.02.2015**
(21) Application number: 12178765.9
(22) Date of filing: 31.07.2012
(51) Int. Cl.: B01D 53/14, C07C 309/44, C07D 233/58, C07C 303/32, B01D 53/18, B01D 53/76, B01D 53/78

(54) **Ionic liquids for selective sulfur dioxide absorption**
Ionische Flüssigkeiten für die selektive Schwefeldioxidabsorption
Liquide ionique pour l'absorption sélective de dioxyde de soufre

(43) Date of publication of application: 05.02.2014
(73) Proprietor: LENZING AKTIENGESELLSCHAFT, 4860 Lenzing (AT)
(72) Inventor: Froschauer, Carmen, 6020 Innsbruck (AT); Schottenberger, Herwig, 6082 Patsch (AT); Sixta, Herbert, 4860 Lenzing (AT); Weber, Hedda, K., 8044 Graz (AT)
(74) Representative: Schwarz & Partner

(56) References cited:
- US-A1- 2010 015 040
- US-A1- 2011 223 084
- US-A1- 2012 042 778
- HUANG ET AL: "Tuning ionic liquids for high gas solubility and reversible gas sorption", JOURNAL OF MOLECULAR CATALYSIS A: CHEMICAL, ELSEVIER, AMSTERDAM, NL, vol. 279, no. 2, 24 December 2007 (2007-12-24), pages 170-176, XP022401241, ISSN: 1381-1169
- ARMSTRONG D W ET AL: "IONIC LIQUIDS AS MATRIXES FOR MATRIX-ASSISTED LASER DESORPTION/IONIZATION MASS SPECTROMETRY", ANALYTICAL CHEMISTRY, AMERICAN CHEMICAL SOCIETY, US, vol. 73, no. 15, 1 August 2001 (2001-08-01), pages 3679-3686, XP001156233, ISSN: 0003-2700, DOI: 10.1021/AC010259F

## Description

The present invention relates to liquid salt compositions and their use as specific gas absorbing media for sulfur dioxide (SO₂). The invention further relates to a method for the synthesis of such compositions. Finally, the invention relates to a device employing the compositions for the selective absorption of SO₂.

Sulfur dioxide (SO₂) is widely used as a valuable intermediate in chemical production and as a key reactant in pulping processes. However, when originating from flue gas emissions, SO₂ fatally contributes to environmental pollution as the main source of acid rain. Flue gas desulfurization (FGD) is therefore an important field of combustion technology, but classic FGD includes various disadvantages, like the generation of large amounts of caustic waste that needs to be disposed, or the volatilization of solvents (Huang, J.; Riisager, A.; Berg, R.W.; Fehrmann, R. J. Mol. Cat. A: Chemical 2008, 279, 170-176). At present the most attractive approaches for SO₂ gas separation are pressure swing absorption (PSA) or temperature swing absorption (TSA) technologies, which are energy saving compared to flue gas desulfurization processes and avoid by-products (S.M. Jeong; S.D. Kim, Ind. Eng. Chem. Res. 2000, 39, 1911).

Metal organic frameworks (MOFs), a new class of porous adsorbent materials, recently have been modified for toxic gas separations (cyanogen chloride and sulfur dioxide), but even such sophisticated systems are suffering from a detrimental competitive uptake of water in humid environment (Glover, G.T.; Peterson, G.W.; Schindler, B.J.; Britt, D.; Yaghi, O.; Chem. Eng. Sci., 2011, 66, 163-170).

It is known in the art to use molten salts exhibiting a melting point below 100°C, widely recognized as ionic liquids (ILs), and preferably room temperature ionic liquids (RTILs) as sorbents for a variety of gases (Anderson, J.L.; Dixon, J.K.; Brennecke, J.F.; Acc. Chem. Res., 2007, 40, 1208-1216). US 6,579,343 describes the energetically advantageous removal of CO₂ from natural gas by means of ILs containing BF₄⁻ and PF₆⁻ as common IL-anions.

ILs, and especially RTILs have recently been intensely investigated for their potential utilization in flue gas separations as reusable and environmentally friendly alternative (Heldebrant, D.J; Yonker, C.R.; Jessop, P.G.; Phan, L., Chem. Eur. J. 2009, 15, 7619-7627). When ILs are used for gas capture, they are capitalizing on their non-volatile nature, preventing any co-condensation upon gas recovery (Davis, J.H., Jr.; WO 2008/122030 A2).

The mechanistic details of SO₂-uptake in ILs are still under dispute, since gas sorption processes are in general attributable to physical interaction with no chemical reactions or absorption processes involving both phenomena, physical and chemical interaction as well (Shiflett, M.B.; Yokozeki, A.; Ind. Eng. Chem. Res., 2010, 49, 1370-1377).

A widely recognized reference system is the (hygroscopic) IL tetramethylguanidinium (TMG) lactate (Wu, W.; Han, B.; Gao, H.; Liu, Z.; Jiang, T.; Huang, J. Angew. Chem. Ind. Ed. 2004, 43, 2415-2417). Unfortunately, due to the inherent carboxylate-basicity, and the very hydrophilic character of its hydroxy acid-derived anionic moiety, TMG lactate is collapsing to an aqueous system upon prolonged exposure to ambient air/sulfur dioxide under gradual precipitate formation. As expected, the final product could be identified as TMG hydrogen sulfite, thus disqualifying this lactate and related ILs as technically useful, reversible scrubbing agents for acid flue gas constituents.

Amino acid derived ILs containing carboxylates as counter anions have been shown to absorb CO₂ in an equimolar manner as well (Zhang, J.; Sun, J.; Zhang, X.; Zhao, Y.; Zhang, S.; Greenhouse Gas Sci Technol. 2011, 1, 142-159). However, they are likewise suffering from deliquescence, and as a result of it, technically irreversible chemisorptive conversions of acid gases into carbonates and sulfites by aqueous alkaline conditions.

On the other hand, if hydrophilicity is circumvented by applying long chain alkyl substituents on the respective cationic hetero-onium counter ions, the resulting high molecular weight of the scrubbing ILs is rendering nominal high molar absorption ratios meaningless because of a significantly lowered actual absorptivity in terms of weight per weight ratio (Gurkan, B.E.; de la Fuente, J. C.; Mindrup, E.M.; Ficke, L.E.; Goodrich, B.F.; Price, E.A.; Schneider, W.F.; Brennecke, J.F.; J. Am. Chem. Soc. 2010, 132, 2116-2117).

In this context, the extremely high solubility of SO₂ in ether functionalized imidazolium mesylates is attributed to side chain/anion combined interactions, featuring an increasing SO₂ solubility with increasing number of tethered ether oxygen atoms (Hong, S.Y.; Im, J.; Palgunadi, J.; Lee, S.D.; Lee, J.S.; Kim, H.S.; Cheong, M.; Jung, K.-D.; Energy Environ. Sci. 2011, 4, 1802-1806). Again, the resulting inherent dual water affinity of cationic and anionic constituents in combination with the higher molecular weight of such purely mole-fraction-optimized scrubbing ILs is restricting their practical use for process relevant applications due to their increased hygroscopicity in combination with reduced nominal gas absorptivity with respect of weight percentages.

As outlined above, the concomitant uptake of adventive moisture under realistic conditions of flue gas scrubbing is in general a severe drawback of hydrophilic ILs, despite their widely appraised high affinity for CO₂ and SO₂. Even though greenhouse gas absorption is enhanced by the basic character of a particular IL (Revelli, A.-L.; Mutelet, F.; Jaubert, J.-N.; J. Phys. Chem. B 2010, 114, 8199-8206), basicity and hydrophilicity are disfavoring or preventing any selectivity. This also holds true for the recently introduced azole anion-based ILs containing the conjugated bases of imidazole or tetrazole (Wang, C; Cui, G.; Luo, X.; Li, H.; Dai, S.; J. Am. Chem. Soc. 2011, 133, 11916-11919).

A further possible pathway of chemisorption, as already demonstrated for carbon dioxide in neat 1,3-dialkylimidazolium acetate ILs (Gurau G.; Rodriguez,H.; Kelley, S.P.; Janiczek, P.; Kalb, R.S.; Rogers, R.D.; Angew. Chem. Int. Ed. 2011, 50, 12024-12026), is the formation of in situ generated nitrogen-heterocyclic-carbenes (NHC-carbenes) by removal of the acidic proton at position C(2)-H of imidazolium cations, followed by the known formation of imidazolium-carboxylate zwitterions. However, since both, NHC-carbenes and imidazolium carboxylates are known to be sensitive to moisture (back-reaction to 1,3-dialkylimidazolium and, casually, liberation of volatile acetic acid), a technical implementation of any respective sequestration process for carbon or sulfur is unfeasible, since also NHC-adducts of SO₂ have been described (Denk, M.K.; Hatano, K.; Lough, A.J.; Eur. J. Inorg. Chem., 2003, 2, 224-231).

Dialkylimidazolium and TMG tetrafluoroborates (Ren, S.; Hou, Y.; Wu, W.; Liu, Q.; Xiao, Y.; Chen, X. J. Phys. Chem B, Letters 2010, 114, 2175-2179) or triflimides (Huang, J.; Riisager, A.; Berg, R.W.; Fehrmann, R. J. Mol. Cat. A: Chemical 2008, 279, 170-176) were among the first intrinsically base-free composited ILs to be recognized to reversibly absorb SO₂ in molar stoichiometric ratios. However, they can hardly be classified as task-specific ILs (TSILs).

In general, the average solubility of SO₂ and CO₂ in ILs lies within about the same order of magnitude. Due to the remarkably high solubility of SO₂ in ILs (and especially in triflimide-based ILs), even with the low partial pressures of SO₂ found in industrial flue gases, it may be possible to remove both SO₂ and CO₂ in a single processing step, while still enabling reuse of the IL (Anderson, J.L.; Dixon, J.N. K.;. Maginn, E.J.; Brennecke, J.F.; J. Phys. Chem. B Letters, 2006,110, 15059-15062). On the other hand, under certain technical circumstances, this lack of selectivity can be considered a general application limiting disadvantage of scrubbing ILs described so far.

When solvents containing molecular compositions with atoms bearing basic lone pairs (e.g. aqueous liquid amines), are applied for the chemical confining of acidic gases like CO₂ and SO₂, it is likely that any formation of the respective ammonium carbonates or bisulfites is an unwanted reaction and will result in the hardly reversible cumulation of such salts as the final products of scavenging. Such reactions are the separation prohibiting factor, regardless whether the basic moieties are incorporated in non volatile functionalized ILs, e.g. taurinates, or introduced as separate chemical additives.

Even for entirely anhydrous systems, with a matching affinity, especially for the Lewis-acidic flue gases CO₂ and SO₂, in particular also for functionalized ILs with covalently incorporated nucleophilic lone pairs (Lewis bases), an inherently unselective simultaneous chemical capture of both gases has to be coped (formation of carbamates and sulfamates respectively). In analogy, carbamate formation mediated by ILs containing aprotic heterocyclic anions (AHAs), such as pyrrolide- or pyrazolide, has been reported recently, and suggested for CO₂ capture and stripping chemistry by means of temperature swing cycles (Gurkan, B.; Goodrich, B.F.; Mindrup, E.M.; Ficke, L.E.; Massel, M.; Seo, S.; Senftle, T.P; Wu, H.; Glaser, M.F.; Shah, J. K.; Maginn, E. J.; Brennecke, J. F.; Schneider; W. F.; J. Phys. Chem. Lett., 2010, 1, 3494-3499). However, no concerns regarding potential co-capturing of acidic flue gases have been argued for ILs comprising heterocyclic anions (Schneider; W. F.; Brennecke, J. F.; Maginn, E. J.; Mindrup, E.M.; Gurkan,B.; Price, E.; Goodrich, B.; WO 2011/056895).

For practical use it is difficult to find a material for reversible and selective absorption of SO₂ (Huang, J.; Riisager, A.; Wasserscheid, P.; Fehrmann, R.; Chem. Commun., 2006, 4027-4029). Whenever alkaline (or even neutral) co-scrubbing of CO₂ and SO₂ is intentionally implemented in a process step for separations of both of these polar acidic flue gases from other flue gas constituents which exhibit a very low solubility in ILs (nitrogen, oxygen, carbon monoxide), a final co-condensate of expelled CO₂/SO₂ mixtures, would again require selective scrubbers in order to replace energy consuming classical, or IL-based temperature swing cycles (Scialdone, M.A.; US 2011/0223084 A).

US 2012/0042778 A1 discloses SO₂ absorbents comprising ionic liquids. US 2010/0015040 A1 discloses a method for separation and recycling of pure sulphur dioxide from a gaseous mixture in the iodine-sulfur cycle using an ionic liquid. Armstrong et al. (Anal. Chem. 2001, 73, 3679 - 3686) discloses different ionic liquids as matrixes for Matrix-Assisted Laser Desorption / Ionization Mass Spectrometry.

Accordingly, all sorption materials used so far for SO₂ have certain drawbacks.

It is therefore an object of the present invention to provide a compound or mixture as scrubbing agent that is a highly selective absorbent for SO₂. In addition, absorption of SO₂ should be reversible so that the absorbent can be used for several absorption/desorption cycles. Further, this absorbent should be insensitive to moisture. The molecular weight of the absorbent should also be small in order to achieve a high absorption ratio per kg of absorbent. Finally, such an absorbent should be easily available by co-opting commercially available commodity chemicals as starting materials, and assembling them in quick, high yielding and atom efficient reactions.

These objects are solved by a of formula [C⁺] [A⁻], wherein
[A⁻] is 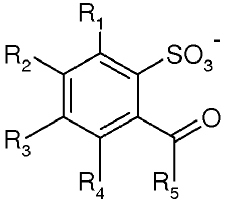 and
[C⁺] is 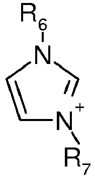 , wherein

| | |
|---|---|
| R₁, R₂, R₃, R₄ | are independently -H; -C₁ to -C₁₂ -alkyl or alkenyl; -OH; -SH; -CN; - F; -Cl; -Br; -I; |
| R₅ | is -H, -C₁ to -C₁₂ -alkyl or alkenyl |
| R₆ | is -C₁ to -C₁₂ -alkyl or alkenyl |
| R₇ | is -C₁ to -C₁₂ -alkyl or alkenyl |

In a preferred embodiment the residues R₁, R₂, R₃, R₄, and R₅ are independently H, -C₁ to - C₁₂ -alkyl or alkenyl.

In one preferred embodiment the residues R₁, R₂, R₃, R₄, and R₅ are H, thus, [A⁻] being

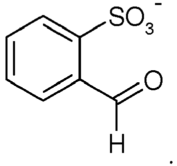

Compounds with this anion are easily available.

Another preferred embodiment is characterized in that R₆ is butyl or allyl and R₇ is methyl. In this case [C⁺] is 1-butyl-3-methylimidazolium and 1-allyl-3-methylimidazolium, respectively.

The described compounds are ionic liquids. As mentioned in the introduction and according to the understanding of the present invention, an ionic liquid is a molten salt exhibiting a melting point below 100°C. More specifically, the compounds according to the present invention are preferably room temperature ionic liquids (RTILs), that are salts exhibiting a melting point of not more than 30 °C, preferably not more than 25 °C.

In one embodiment of the invention, absorption enhancers for SO₂ are admixed to the compound. An absorption enhancer is a compound that increases the amount or rate of absorption of SO₂. Adsorption enhancers may also be constituted of ionic liquids or ionic liquid-related salts. Hence, the invention relates to a mixture of the compound described above and an additional absorption enhancer for SO₂. In a preferred embodiment the absorption enhancer is an ionic liquid or an ionic liquid related salt. An ionic liquid related salt is to be understood as an ionic compound wherein one ion (cation or anion) is a known cation or anion in ionic liquids.

The ionic liquid admixed to the compound is preferably of formula [Q⁺]_{b}[B^{b-}], wherein [Q⁺] is selected from the group of 2-hydroxyethanesulfonate, p-xylene-2-sulfonate or a combination thereof.

The invention further relates to a method for the synthesis of the compound mentioned above. The method comprises treating a compound [C⁺][Y⁻] with a compound [Z^{z+}][A⁻]_{z}. The reaction product [C⁺] [A⁻] is separated from the reaction mixture by suitable means. In an embodiment of the invention
(a) [Y⁻] is selected from the group of BF₄⁻, Cl⁻, Br⁻, I⁻, or OH⁻ and
(b) [Z^{z+}] is selected from the group of H⁺, alkali metal ion, and alkaline earth metal ion.

In one embodiment sodium 2-formylbenzenesulfonate 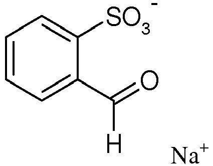 is used for [C⁺][Y⁻].

In one embodiment [Z^{z+}][A⁻]_{z} is 1-butyl-3-methylimidazolium chloride and in another embodiment it is 1-allyl-3-methylimidazolium chloride.

It is possible to place [C⁺][Y⁻] and [Z^{z+}][A⁻]_{z} in a suitable vessel alone or together with an organic solvent. This mixture is agitated. The reaction process may be monitored, e.g. by watching salt precipitation of [Z^{z+}][Y⁻]_{z} or analysing the reaction mixture by suitable means (e.g. IR, NMR, etc).

The formed [C⁺] [A⁻] is separated from the reaction mixture by suitable separation techniques. These include filtering, extraction, distillation etc. In one embodiment formed [Z^{z+}][Y⁻]_{z} is filtered off and the remaining solvent (including [C⁺] [A⁻]) evaporated to isolate compound [C⁺] [A⁻]. The obtained compound is very pure so no further cleaning step is necessary. However, additional cleaning steps such as distillation, washing with suitable solvent(s), exposing to vacuum etc. may be applied.

The invention of course also relates to the use of the compound or mixture for the selective absorption of SO₂. As the high selectivity for SO₂-absorption is mainly due to the anion, according to the invention the use of an ionic liquid comprising a compound of formula [C⁺] [A⁻], wherein
[A⁻] is 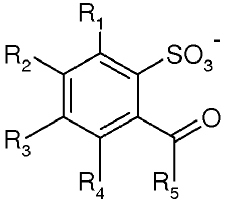 and

| | |
|---|---|
| R₁, R₂, R₃, R₄ | are independently -H; -C₁ to -C₁₂ -alkyl or alkenyl; -OH; -SH; -CN; - F; -Cl; -Br; -I; |
| R₅, | is -H, -C₁ to -C₁₂ -alkyl or alkenyl |

is suggested.

In accordance with the findings already described above the preferred use includes that the compound of [C⁺] [A⁻] is an ionic liquid according to one of claims 1 to 4 or a mixture according to one of claims 5 to 7 for the absorption of SO₂.

Also the invention relates to an ionic liquid of formula [E⁺] [A⁻], wherein [A⁻] is 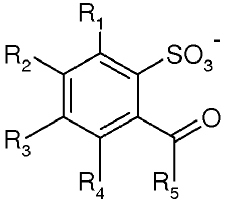 and
[E⁺] is a suitable cation, wherein

| | |
|---|---|
| R₁, R₂, R₃, R₄ | are independently -H; -C₁ to -C₁₂ -alkyl or alkenyl; -OH; -SH; -CN; - F; -Cl; -Br; -I; |
| R₅ | is -H, -C₁ to -C₁₂ -alkyl or alkenyl |

In a preferred embodiment the residues R₁, R₂, R₃, R₄, and R₅ are independently H, -C₁ to - C₁₂ -alkyl or alkenyl.

In one preferred embodiment the residues R₁, R₂, R₃, R₄, and R₅ are H, thus, [A⁻] being

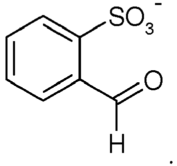

The invention further relates to a device for the absorption of SO₂, comprising a reservoir, an inlet and an outlet for flue gas, wherein the reservoir comprises a compound or a mixture as mentioned above. The idea is that flue gas containing SO₂ enters the device via the inlet. After entering the device, SO₂ is contacted with the compound/mixture from the reservoir. There are several possibilities of how the flue gas may be contacted with the compound/mixture. In one embodiment the inlet is connected directly with the reservoir in such a manner that flue gas flowing into the device is passed through the liquid compound/mixture in the reservoir.

Other embodiments employ a nebulisation apparatus, a film forming apparatus, a foam forming apparatus or combinations thereof. All such apparatuses aim to increase the surface of the compound/mixture in order to provide a larger area for absorption of SO₂. A nebulisation apparatus forms small droplets of the compound, a film forming apparatus creates a film of the compound/mixture on a surface whereas a foam forming apparatus creates a porous structure with a large surface.

In a preferred embodiment the device comprises a heating device for the compound/mixture. The device for absorption of SO₂ may further comprise an absorption region in which the compound is exposable to SO₂ and a desorption region where SO₂ may be desorbed, wherein a heating device is present in the desorption region.

As mentioned above, the compound/mixture absorbs SO₂ selectively in disfavour of CO₂ and reversibly. SO₂ can be desorbed by increasing the temperature of the compound binding SO₂. Hence, the device may comprise a heating device for desorbing SO₂ and thus regenerating the compound/mixture. Preferably, the ionic liquid according to the invention should have a lower temperature in the absorption area whereas it should have a higher temperature in the desorption area. In order to implement this finding into the device, the device should have an area for absorption, an area for desorption which is distant to the area for absorption, whereas the heating device is only placed in the desorption area.

Preferably, the device is coupled with a vacuum air unit to increase the rate of SO₂ desorption for regeneration of the compound. In the presence of an absorption region for SO₂ and a desorption region for SO₂ it is preferred that the vacuum air unit is coupled to the desorption region.

Further details and advantages of the invention are presented below. The figures and the description of the figures highlight some advantages and details.
- Fig. 1: shows an SO₂ sorption profile of different sorbents for SO₂.
- Fig. 2: shows the selectivity of 1-butyl-3-methylimidazolium 2-formylbenzenesulfonate for SO₂ versus CO₂.
- Fig. 3: shows a DSC of 1-butyl-3-methylimidazolium 2-formylbenzenesulfonate.
- Fig. 4: shows the ability for regeneration (absorption/desorption) of two compounds according to the invention compared to an absorption enhancer and prior art absorbents.
- Fig. 5: shows the SO₂ desorption rate of 1-butyl-3-methylimidazolium 2-formylbenzenesulfonate with absorbed SO₂.
- Fig. 6: shows the absorption fraction of SO₂ / 1-butyl-3-methylimidazolium 2-formylbenzenesulfonate.
- Fig. 7: shows an example for a device according to the invention.

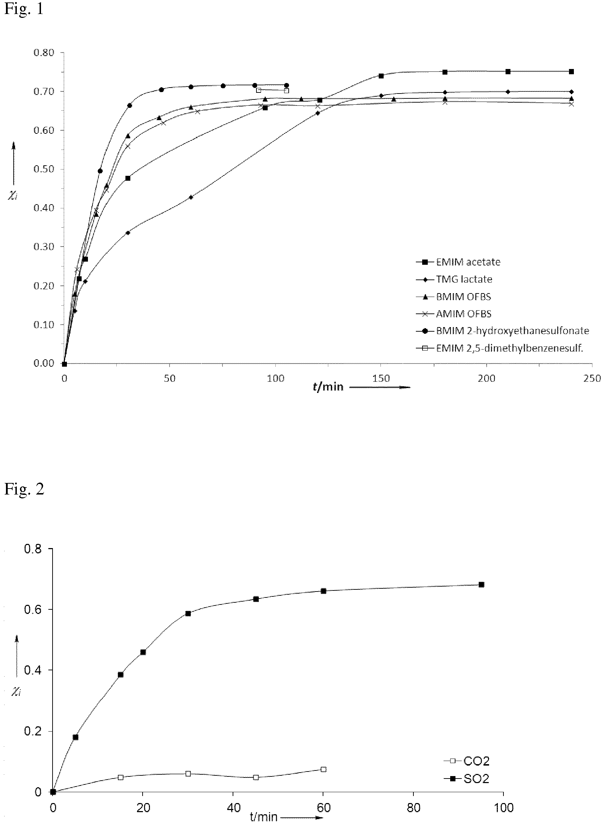

Fig. 1 shows SO₂ absorption profiles as mole fraction χᵢ (mole SO₂ per mole sorbent; ordinate) of SO₂ in solvents as a function of time (abscissa). 1-butyl-3-methylimidazolium 2-formylbenzenesulfonate (= 1-butyl-3-methylimidazolium ortho-formylbenzenesulfonate, abbrev.: BMIM OFBS, full triangles in Fig. 1) and 1-allyl-3-methylimidazolium 2-formylbenzenesulfonate (AMIM OFBS, X in Fig. 1) are compounds according to the invention. BMIM 2-hydroxyethanesulfonate, full squares, is an absorption enhancer. The remaining examples relate to prior art: EMIM acetate (full squares) = 1-ethyl-3-methylimidazolium acetate; TMG lactate (full rhombus) = tetramethylguanidinium lactate; EMIM (1-ethyl-3-methylimidazolium) 2,5-dimethylbenzenesulfonate (open squares).

Fig. 2 shows the selectivity of 1-butyl-3-methylimidazolium 2-formylbenzenesulfonate towards SO₂ versus CO₂ absorption profiles shown as mole fraction χᵢ (mole SO₂ per mole 1-butyl-3-methylimidazolium 2-formylbenzenesulfonate; ordinate) as a function of time (abscissa). As can be clearly seen, the uptake is more than one order of magnitude more selective for of SO₂ (full squares) than for CO₂ (open squares).

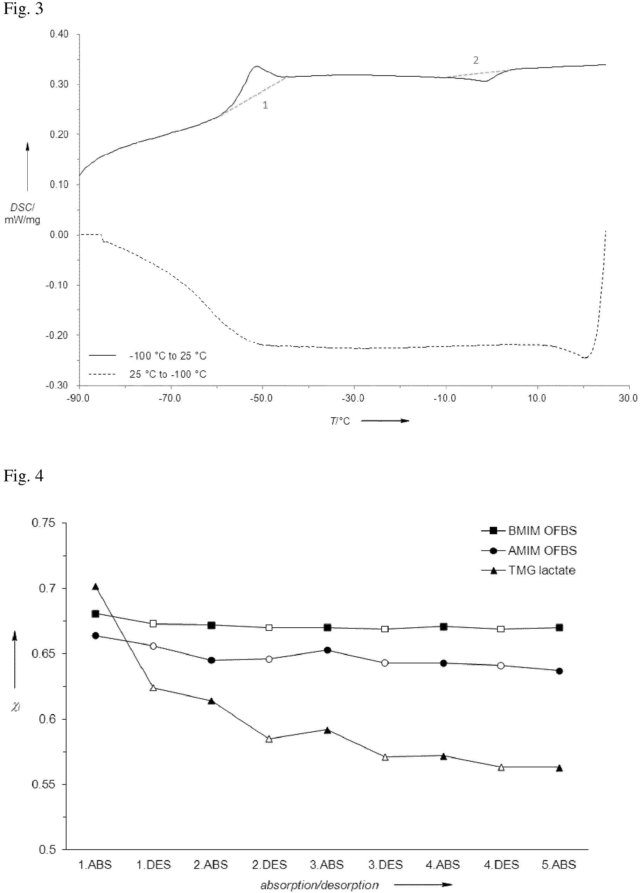

Fig. 3 shows differential scanning calorimetry (DSC) of 1-butyl-3-methylimidazolium 2-formylbenzenesulfonate, demonstrating the liquidus range of neat 1-butyl-3-methylimidazolium 2-formylbenzenesulfonate. As can be seen there is no solidification or glass transition above -40° C. Hence it is proven that 1-butyl-3-methylimidazolium 2-formylbenzenesulfonate is an ionic liquid even far below room temperature. Reference 1: area 2.38 J/g, peak: -51.3°C, onset: -56.7 °C; reference 2: area -0.47 J/g, peak: -2.0°C, onset: -5.0 °C.

Fig. 4 shows repeated SO₂ absorption/desorption cycles. The absorbed and desorbed mole fraction in each step is shown. 1-butyl-3-methylimidazolium 2-formylbenzenesulfonate (squares) and 1-allyl-3-methylimidazolium 2-formylbenzenesulfonate (circles) show a stable SO₂ uptake capacity with no remarkable change over several cycles of desorption and absorption. The comparative example TMG lactate (triangles) show a high fraction of SO₂ absorption in the first cycle but due to chemical conversion, the desorbed amount is rather poor and hence the mole fraction over several cycles decreases significantly making TMG lactate a poor candidate for reversible SO₂ absorption/desorption.

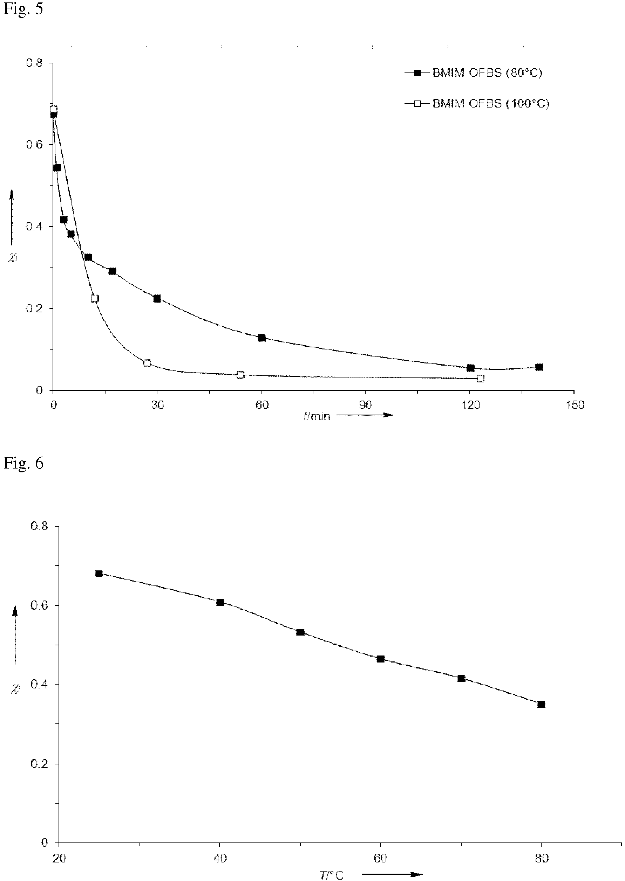

Fig. 5 shows the temperature dependence of the desorption of SO₂ from 1-butyl-3-methylimidazolium 2-formylbenzenesulfonate again as mole fraction SO₂ per 1-butyl-3-methylimidazolium 2-formylbenzenesulfonate. As can be seen (and as expected), desorption at 80 °C (full squares) takes more time than at 100 °C (open squares). Hence for fast regeneration of the ionic liquid higher temperatures are preferred.

Fig. 6 shows that the capacity for absorbing SO₂ is higher in 1-butyl-3-methylimidazolium 2-formylbenzenesulfonate at low temperatures than at higher temperatures. At room temperature the mole fraction is approximately around 0.7 moles SO₂ per mole 1-butyl-3-methylimidazolium 2-formylbenzenesulfonate whereas at 80 °C it decreases to around 0.3 indicating - as to be expected - that the equilibrium between absorption / desorption is shifted towards desorption at higher temperatures. Hence, for an SO₂ absorption device preferably, the ionic liquid according to the invention should have a lower temperature in the absorption area whereas it should have a higher temperature in the desorption area.

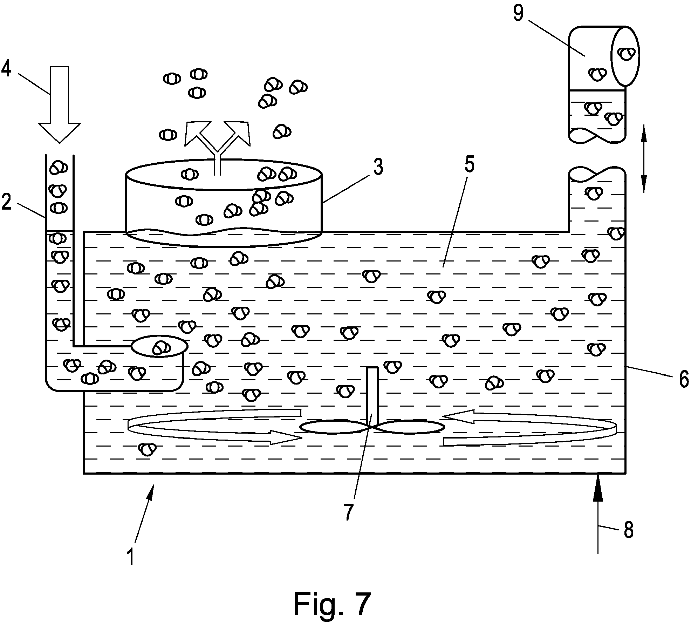

Fig. 7 shows a schematic principle of continuous sour gas refining by bubbling off CO₂ under SO₂ retainment, with simultaneous (optionally heat assisted) stripping off SO₂ by evacuation from the non volatile IL in a conjugated scrubbing compartment. The shown device 1 comprises an inlet 2 for flue gas 4 and an outlet 3. The compound 5 is in a reservoir 6 in form of a container. A stirring device 7 stirs the ionic liquid and it creates also a temperature gradient in the ionic liquid since the heating device 8 is in the region distant to the inlet 2 so SO₂ can absorb at lower temperatures near the inlet 2 and desorb near the vacuum air device 9 distant to the inlet 2.

Basically the selective sorption mechanism is based on a functional discrimination between CO₂ and SO₂. This discrimination relies on the amphoteric character of the latter, actually by addressing the remaining lone pair of the sulfur atom in its tetravalent oxidation state.

One of the findings of the present invention is that the anionic constituents of selective scrubbing ILs should be no conjugated bases of weak acids (preferably carboxylate-free, carbonate-free, and azolate-free in order to avoid the simultaneous formation and segregation of bisulfite and bicarbonate, or the simultaneous formation of NHC-carbene adducts, or carbamates and sulfamates during gas capture cycles, unfavorably accompanied by deliquescence).

Rather, they should preferably
- contain only neutral, base-free moieties precluding any unselective formation of strong Lewis acid-base adducts with acidic greenhouse gases
- be halide-free (non corrosive, non hygroscopic)
- be non hygroscopic, stable and recyclable
- rely on low cost industrial bulk chemicals
- be liquid below room temperature (higher gas absorptivity, capable of swing cycles
- be physisorptive, with reversible absorption mechanisms and no chemical derivation
- be inert to phase collapsing behavior by cumulation of ambient moisture

In order to investigate associative actions of SO₂ with electrophilic sites in the cybotactic environment of ILs, a unique and affordable family of RTILs, based on the newly introduced anion 2-formylbenzenesulfonate was prepared, and the solubility of SO₂ in this ILs was determined. Their performance in absorption-desorption cycles for SO₂ turned out to be superior in comparison to all other systems described so far with respect to moisture-sensitivity and reversibility, selectivity, and sorption capacity.

Surprisingly and unexpectedly it was found that the intentional absorption mechanism of SO₂ in 2-formylbenzenesulfonates is not accompanied by any eventual formation of bisulfite adducts, but instead, it is operative without any simultaneous uptake of water. This can be confirmed unequivocally by NMR- und infrared spectroscopy (data not shown). A regular (chemisorptive) aldehyde-bisulfite addition reaction would require equimolar amounts of water, and the products would exhibit all the characteristics of the resulting arene-substituted hydroxymethanesulfonates.

Only with highly selective ILs as gas absorbers, a specific partial capacity breakdown, usually even induced by ambient air (N₂), is avoidable. Despite the unparalleled gas absorption behavior of formylbenzenesulfonate-based ILs, namely their high capacity and extraordinary selectivity between SO₂ and CO₂ (Fig. 2), no irreversible sulfur sequestration by bisulfite adduct formation with the formyl substituent takes place. The specifically affinity-tailored new family of affordable, neutral, non corrosive, non volatile, non deliquescent ILs are easily accessible by a one step IL synthesis (salt metathesis), thus providing fully reversible and recyclable SO₂-selective scrubbing systems.

The high selectivity of the new ILs even would allow for gas refining by simultaneous bubbling off CO₂ under ambient (atmospheric) pressure from a conjugated fluid-charged scrubbing compartment, where the selectively retained SO₂ may be pumped off by concomitant evacuation from above a vertical hydrostatic piping distance (required for the IL-density related Torricelli-vacuum, Fig. 7). The new systems would also allow for the additional implementation of (even combined) swing cycles (PSA and TSA respectively).

The new ILs are liquid at room temperature and far below, and are therefore highly compatible with recent methods of (optional) surface optimization like electrospraying or nebulization by ultrasound agitation. Likewise, the removal of captured gases may be achieved by ultrasonication as well.

In their entirety of properties, and in particular their absorption profiles, the newly introduced ILs are, with respect to the criteria required for technical sour gas capturing, superior to any previously described ILs, as well as to all competitive principles based on the utilization of volatile containing fluids or slurry compositions.

Without any chemical alteration of the ILs during repeated load/recovery cycles, the newly disclosed ILs exhibit strikingly high affinities towards SO₂. In parallel, because of the absence of basic functionalities, CO₂ is retained only negligibly, thus furnishing a fundamental technical achievement.

Consequently, the highly demanded sequestration (and separation) of flue gas constituents in separate consecutive steps should be a feasible chemical process, e.g. to facilitate recirculating steps of a specific (optionally excess) reactive off-gas fraction into a (continuous) chemical process.

A summative overview of disadvantages of anions incorporated in predescribed ILs disclosed for flue gas scrubbing, and related processes for gas sorption are common knowledge to persons skilled in the art, but may be retrieved as well in the citations given in this document.

### BF4⁻, tetrafluoroborate-based ILs, TSILs, functionalized ILs, and related salts:

Hydrolysis in the presence of adventive moisture (liberation of toxic hydrogen fluoride), usually miscible with water in any ratio, hygroscopic or deliquescent; lack of CO₂/SO₂-selectivity; competitive uptake of ambient nitrogen in disfavor of SO₂, lowering the saturation equilibrium to a level of below 0.1 (molar ratio of SO₂ to ILs).

### PF₆⁻, hexafluorophosphate-based ILs, TSILs, functionalized ILs, and related salts:

Long term hydrolysis in the presence of adventive moisture (liberation of HF and conversion to less hydrophobic mixtures by formation of very corrosive, hydrophilic derivatives of phosphoric acid); higher melting points in comparison with other suitable, weakly coordinating anions of hydrophobic ILs; lack of CO₂/SO₂-selectivity.

### N(tf)₂⁻, triflimide-based ILs, TSILs, functionalized ILs, and related salts:

Costly, very low CO₂/SO₂-selectivity (most ILs claimed by various patent applications); competitive uptake of ambient nitrogen in disfavour of SO₂, lowering the saturation to a level of below 0.1 (molar ratio of SO₂ to ILs); lack of CO₂/SO₂-selectivity; extremely low solubility of cooperative sulfonate-based salts, especially zwitterions; potential liberation of extremely volatile, acidic bis(trifluroromethylsulfonyl)amine due to protonation of triflimide anion by sulfuric acid which may be formed in the presence of ambient oxygen (moist process air) due to oxidation of SO₂ to SO₃ and subsequent hydrolysis to sulfuric acid.

### Amino-functionalized cations, or anions, or zwitterions incorporated in ILs disclosed for flue gas scrubbing:

Usually miscible with water in any ratio, hygroscopic or deliquescent; lack of CO₂/SO₂-selectivity.

### Carboxylate or carbonate anions incorporated in ILs disclosed for flue gas scrubbing:

Lack of CO₂/SO₂-selectivity, potential deliquescent collapse induced by formation of hydrophilic salts; chemisorptive absorption mechanism, formation of carbonates, and bisulfites due to alkaline reaction upon uptake of adventive moisture by real process effluent streams.

### Heteroaromatic anions, aprotic heterocyclic anions (AHAs):

Lack of CO₂/SO₂-selectivity, potential deliquescent collapse induced by formation of hydrophilic salts; chemisorptive absorption mechanism, formation of carbonates, and bisulfites due to alkaline reaction upon uptake of adventive moisture, or, without adventive humidity, formation of carbamates and sulfamates, respectively.

Conclusively, the critical application-restricting disadvantages of the hitherto most promising tetrafluoroborate-, triflimide-based, and related ILs, so far targeted as absorbers of polar gases, is the remarkable absorptivity breakdown caused by the presence of ambient air constituents (a common technical situation), namely humidity and nitrogen gas.

### Examples:

Examples 1 and 2 describe methods for synthesizing compounds according to the invention. Examples 3 and 4 describe two absorption enhancers and their synthesis.

### Example 1: 1-Butyl-3-methylimidazolium 2-formylbenzenesulfonate:

Sodium 2-formylbenzenesulfonate and an equimolar amount of 1-butyl-3-methylimidazolium chloride were placed in a round bottom flask and suspended in acetone by stirring at room temperature for three days. Upon stirring, sodium chloride starts to precipitate, which was then filtered with a sintered glass frit. The solvent was evaporated by means of a rotary evaporator and finally the yellow liquid was dried vial oil pump vacuum while keeping the temperature at 80°C by means of an oil bath.
¹H NMR (DMSO-d₆): δ 0.82 (3H, t, 7.4 Hz), 1.18 (2H, m, 7.5 Hz), 1.70 (2H, m, 7.4 Hz), 3.83 (3H, s), 4.12 (2H, t, 7.2 Hz), 7.50 (1H, t,7.4 Hz), 7.61 (1H, t, 7.5 Hz), 7.69 (1H, s), 7.75 (1H, s), 7.76 (1H, s), 7.85 (1H, d, 7.6 Hz), 9.14 (1H, s), 10.93 (1H, s) ppm.
¹³C NMR (DMSO-d₆): δ 13.20, 18.75, 31.35, 35.68, 48.47, 122.24, 123.56, 126.51, 126.80, 129.36, 132.55, 136.54, 149.69, 193.67 ppm.
IR (neat): 3145, 3099, 2960, 2933, 2873, 1687, 1585, 1570, 1464, 1396, 1338, 1298, 1259, 1188, 1132, 1080, 1018, 827, 768, 731, 642, 611, 563 cm⁻¹.
Viscosity: 1,65 Pa*s (25 °C)

### Example 2: 1-allyl-3-methylimidazolium 2-formylbenzenesulfonate:

Sodium 2-formylbenzenesulfonate and an equimolar amount of 1-allyl-3-methylimidazolium chloride were placed in a round bottom flask and suspended in acetone by stirring at room temperature for three days. Upon stirring, sodium chloride starts to precipitate, which was then filtered with a sintered glass frit. The solvent was evaporated by means of a rotary evaporator and finally the yellow liquid was dried vial oil pump vacuum while keeping the temperature at 80°C by means of an oil bath.
¹H NMR (DMSO-d₆): δ 3.84 (3H, s), 4,81 (2H,d, 6.0 Hz), 5.27 (2H, m), 5.99 (1H, m, 6.3 Hz), 7.51 (1H, t, 7.5 Hz), 7.62 (1H, t, 7.5 Hz, 7.69 (1H, d, 6.8 Hz), 7.77 (1H, d, 7.6 Hz), 7.87 (1H, d, 7.7 Hz), 9.12 (1H, s), 10.95 (1H, s) ppm.
¹³C NMR (DMSO-d₆): δ 35.78, 50.78, 120.31, 122.29, 123.74, 126.60, 126.83, 129.46, 131.63, 132.58, 133.25, 136.65, 149.58, 193.70 ppm.

### Example 3: 1-butyl-3-methylimidazolium 2-hydroxyethanesulfonate (non volatile modifier, absorption enhancer):

Sodium 2-hydroxyethanesulfonate and an equimolar amount of 1-butyl-3-methylimidazolium chloride were placed in a round bottom flask and, after ultrasonication for 30 minutes, suspended in acetonitrile by stirring at room temperature for six days. Upon stirring, sodium chloride starts to precipitate, which was then filtered with a sintered glass frit. The solvent was evaporated by means of a rotary evaporator and finally the yellow liquid was dried vial oil pump vacuum while keeping the temperature at 80°C by means of an oil bath. NMR analysis shows a presence of hydroxyethanesulfonate of 83,3% in comparison to the 1-butyl-3-methylimidazolium cation. It can be assumed, that there is still a remarkable amount of chloride present. Changing the solvent to acetone generated no changes to this effect.
¹H NMR (DMSO-d₆): δ 0.80 (3H, t, 7.4 Hz), 1.18 (2H, m, 7.6 Hz), 1.71 (2H, m, 7.4 Hz), 2.67 (1.44 H, t, 7.1 Hz), 3.56. (0.82 H, s), 3.62 (1.45 H, d, 4.0 Hz), 3.85 (3H, s), 4.16 (2H, t, 7.1 Hz), 7.76 (1H, s), 7.84 (1H, s), 9.36 (1H, s) ppm.
¹³C NMR (DMSO-d₆): δ 13.75, 19.30, 31.99, 36.18, 48.95, 54.47, 58.23, 122.84, 124.10, 137.32 ppm.

### Example 4: 1-ethyl-3-methylimidazolium 2,5-dimethylbenzenesulfonate (non volatile modifier, absorption enhancer):

P-xylene-2-sulfonic acid hydrate was added to an equimolar amount of a 1-ethyl-3-methylimidazolium methylcarbonat solution (50% in methanol) in a round bottom flask.
After stirring the solution at room temperature for 2 hours, the solvent was evaporated by means of a rotary evaporator and finally vial oil pump vacuum.
¹H NMR (DMSO-d₆): δ 1.34 (3H, t, 7.3 Hz), 2.22 (3H, s), 2.51 (3H, s), 3.82 (3H, s), 4.15 (2H, q, 7.3 Hz), 7.01 (2H, s), 7.61 (1H, s), 7.71 (1H, s), 7.80 (1H, s), 9.26 (1H, s) ppm.
¹³C NMR (DMSO-d₆): δ 15.05, 19.63, 20.54, 35.50, 44.02, 121.91, 123.48, 127.00, 129.19, 130.72, 132.32, 133.74, 136.49, 145.83 ppm.

### Experimental Part:

The apparatus for the gas absorption consisted of a glass vial filled with 2-3 g of the IL in a water bath.

Continuous stirring of the IL and the water bath was achieved by a magnetic stirrer. The vial was closed with a septum. Two syringe needles were punctured through the septum, one to bubble the flue gas through the IL and one to convey the excess SO₂ into a NaOH solution for binding. The SO₂ flow rate was controlled with a flowmeter purchased from Supelco and was adjusted to about 30 mL/min. The vial filled with the IL was weighed at the beginning of the absorption and then after certain time intervals. The amount of absorbed SO₂ was calculated by the difference of the vial weight before and after absorption. The amount of absorbed SO₂ in ILs versus time was determined at 298.15 K and 1 bar; the equilibrium can be evaluated after 2-3 h. After the run was completed, an aliquot of the sample was removed for NMR and IR analysis to control if the IL has chemically altered by the exposure to SO₂.To study the recovery of SO₂ and the recyclability of the IL, a few gravimetric desorption experiments on ILs saturated with SO₂ were conducted at the temperatures mentioned above. Therefore the vial with the SO₂-saturated IL was opened and put into an oil bath with a magnetic stirrer. The vial was again weighed after defined time periods and the loss of weight versus time was demonstrated.

SO₂ was purchased from Sigma Aldrich with a minimum purity of 99.9% and was used as received.

The ILs were synthesized in our laboratory. 1,1,3,3-tetramethylguanidinium lactate (TMG lactate) was synthesized by direct neutralisation of 1,1,3,3-tetramethylguanidine and lactic acid. 1-Ethyl-3-methylimidazolium acetate (EMIM acetate) was purchased from Iolitec and used as received. 1-Butyl-3-methylimidazolium hexafluorophosphate (BMIM PF₆), 1-butyl-3-methylimidazolium bis(trifluoromethanesulphonyl)imide (BMIM N(tf)₂), cholinium bis(trifluoromethanesulphonyl)imide and 1-butyl-3-methylimidazolium 2-formylbenzenesulfonate (BMIM OFBS) were synthesized.

The ILs were dried under vacuum after the synthesis, but not directly before use for absorption measurements to simulate actual process conditions, which are in effect humid.

## Claims

1. Ionic liquid with a melting point below 100 °C, comprising the ion [A⁻], wherein
[A⁻] is 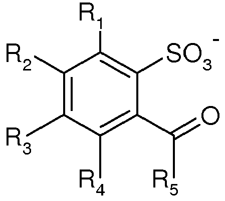 and
| | |
|---|---|
| R₁, R₂, R₃, R₄ | are independently -H; -C₁ to -C₁₂ -alkyl or alkenyl; -OH; -SH; -CN; - F; -Cl; -Br; -I; |
| R₅ | is -H, -C₁ to -C₁₂ -alkyl or alkenyl. |

2. Ionic liquid according to claim 1, wherein the ionic liquid is a compound of formula [C⁺] [A⁻], wherein
[C⁺] is 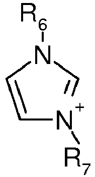 , wherein
| | | |
|---|---|---|
| R₆ | is | -C₁ to -C₁₂ -alkyl or alkenyl |
| R₇ | is | -C₁ to -C₁₂ -alkyl or alkenyl. |

3. Ionic liquid according to claim 1 or claim 2, **characterized in that** R₁, R₂, R₃, R₄, and R₅ are H.

4. Ionic liquid according to any of claims 1 to 3, **characterized in that** R₆ is butyl or allyl and R₇ is methyl.

5. Mixture comprising an ionic liquid according to one of claims 1 to 4 and an absorption enhancer.

6. Mixture according to claim 5, **characterized in that** the absorption enhancer is an ionic liquid or an ionic liquid related salt.

7. Mixture according to claim 6, **characterised in that** the additional ionic liquid or ionic liquid related salt is of formula [Q⁺]_{b}[B^{b-}], wherein [Q⁺] is selected from the group of 2-hydroxyethanesulfonate, p-xylene-2-sulfonate or a combination thereof.

8. A method for the synthesis of an ionic liquid according to one of claims 2 to 4, by treating a compound [C⁺] [Y⁻] with a compound [Z^{z+}] [A⁻]_{z}.

9. A method according to claim 8, wherein
(a) [Y⁻] is selected from the group of BF₄⁻, Cl⁻, Br⁻, I⁻, or OH⁻ and
(b) [Z^{z+}] is selected from the group of H⁺, alkali metal ion, alkaline earth metal ion.

10. Device for absorption of SO₂, comprising a reservoir, an inlet and an outlet for flue gas, wherein the reservoir comprises an ionic liquid according to one of claims 1 to 4 or a mixture according to one of claims 5 to 7.

11. Device according to claim 10, **characterized by** a nebulization, film forming or foam forming device.

12. Device according to claim 10 or claim 11, **characterized in that** it comprises an absorption region in which the ionic liquid is exposable to SO₂ and a desorption region where SO₂ may be desorbed, wherein a heating device is present in the desorption region.

13. Use of an ionic liquid comprising a compound of formula [C⁺] [A⁻], wherein
[A⁻] is 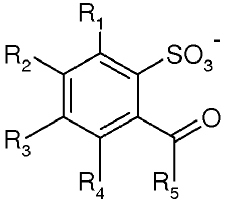 and
| | | |
|---|---|---|
| R₁, R₂, R₃, R₄ | are | independently -H; -C₁ to -C₁₂ -alkyl or alkenyl; -OH; -SH; -CN; - F; -Cl; -Br; -I; |
| R₅, | is | -H, -Cl to -C₁₂ -alkyl or alkenyl |

14. Use according to claim 13, **characterized in that** the compound of [C⁺] [A⁻] is a compound according to one of claims 1 to 4 or a mixture according to one of claims 5 to 7 for the absorption of SO₂.

## Patentansprüche

1. Ionische Flüssigkeit mit einem Schmelzpunkt unter 100 °C, umfassend das Ion [A⁻], wobei
[A⁻] gleich 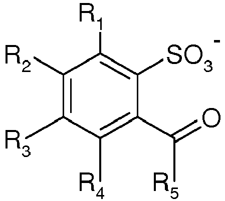 ist und
| | | |
|---|---|---|
| R₁, R₂, R₃, R₄ | sind | unabhängig -H; -C₁ to -C₁₂ -Alkyl oder Alkenyl; -OH; -SH; - CN; -F; -Cl; -Br; -I; |
| R₅ | ist | -H, -C₁ bis -C₁₂ -Alkyl oder Alkenyl. |

2. Ionische Flüssigkeit nach Anspruch 1, **dadurch gekennzeichnet, dass** die ionische Flüssigkeit eine Verbindung der Formel [C⁺] [A⁻] ist, wobei
[C⁺] gleich 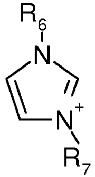 ist, wobei
| | | |
|---|---|---|
| R₆ | gleich | -C₁ bis -C₁₂ -Alkyl oder Alkenyl |
| R₇ | gleich | -C₁ bis -C₁₂ -Alkyl oder Alkenyl ist. |

3. Ionische Flüssigkeit nach Anspruch 1 oder Anspruch 2, **dadurch gekennzeichnet, dass** R₁, R₂, R₃, R₄, und R₅ gleich H sind.

4. Ionische Flüssigkeit nach einem der Ansprüche 1 bis 3, **dadurch gekennzeichnet, dass** R₆ gleich Butyl oder Allyl und R₇ gleich Methyl ist.

5. Mischung, umfassend eine ionische Flüssigkeit nach einem der Ansprüche 1 bis 4 und einen Absorptionsverstärker.

6. Mischung nach Anspruch 5, **dadurch gekennzeichnet, dass** der Absorptionsverstärker eine ionische Flüssigkeit oder ein verwandtes Salz einer ionischen Flüssigkeit ist.

7. Mischung nach Anspruch 6, **dadurch gekennzeichnet, dass** die zusätzliche ionische Flüssigkeit oder verwandte Salz einer ionischen Flüssigkeit eine Verbindung der Formel [Q⁺]_{b}[B^{b-}] ist, wobei [Q⁺] aus der Gruppe 2-Hydroxyethanesulfonat, p-Xylen-2-sulfonat oder einer Kombination daraus ausgewählt ist.

8. Verfahren zur Herstellung einer ionischen Flüssigkeit nach einem der Ansprüche 2 bis 4, umfassend die Behandlung einer Verbindung [C⁺][Y⁻] mit einer Verbindung [Z^{z+}][A⁻]_{z}.

9. Verfahren nach Anspruch 8, **dadurch gekennzeichnet, dass**
(a) [Y⁻] ausgewählt ist aus der Gruppe von BF₄⁻, Cl⁻, Br⁻, I⁻ oder OH- und
(b) [Z^{z+}] ausgwählt ist aus der Gruppe von H⁺, Alkalimetalion, Erdalkalimetallion.

10. Vorrichtung zur Absorption von SO₂, umfassend ein Reservoir, einen Einlass und einen Auslass für Rauchgas, wobei das Reservoir eine ionische Flüssigkeit nach einem der Ansprüche 1 bis 3 oder eine Mischung nach einem der Ansprüche 5 bis 7 umfasst.

11. Vorrichtung nach Anspruch 10, **gekennzeichnet durch** eine Vernebelungs-, Filmbildner- oder Schaumbildnervorrichtung.

12. Vorrichtung nach Anspruch 10 oder 11, **gekennzeichnet durch** einen Absorptionsabschnitt, in welchem die ionische Flüssigkeit SO₂ aussetzbar ist, und einen Desorptionsabschnitt, in welchem SO₂ desorbierbar ist, wobei im Desorptionsabschnitt eine Heizeinrichtung vorhanden ist.

13. Verwendung einer ionischen Flüssigkeit, umfassend eine Verbindung der Formel [C⁺] [A⁻], wobei [A⁻] ist 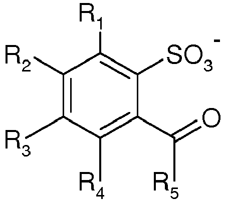 und
| | | |
|---|---|---|
| R₁, R₂, R₃, R₄ | jeweils | unabhängig -H; -C₁ to -C₁₂ -Alkyl oder Alkenyl; -OH; -SH; -CN; -F; -Cl; -Br; -I; sind |
| R₅, | gleich | -H, -C₁ bis -C₁₂ -Alkyl oder Alkenyl ist. |

14. Verwendung nach Anspruch 13, **dadurch gekennzeichnet, dass** die Verbindung [C⁺] [A⁻] eine Verbindung nach einem der Ansprüche 1 bis 4 oder eine Mischung nach einem der Ansprüche 5 bis 8 ist, zur Absorption von SO₂.

## Revendications

1. Liquide ionique ayant un point de fusion inférieur à 100°C, comprenant l'ion [A⁻], dans lequel
[A⁻] est 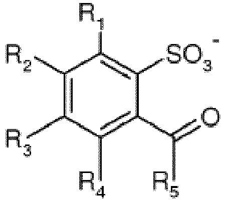 et
| | |
|---|---|
| R₁, R₂, R₃, R₄ | sont indépendamment -H ; -alkyle ou alcényle en C₁ à C₁₂ ; -OH ; -SH ; -CN ; -F ; -Cl ; -Br ; -I ; |
| R₅ | est -H, -alkyle ou alcényle en C₁ à C₁₂. |

2. Liquide ionique selon la revendication 1, dans lequel le liquide ionique est un composé de formule [C⁺] [A⁻], dans laquelle
[C⁺] est 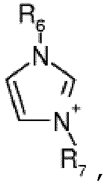 dans laquelle
| | |
|---|---|
| R₆ | est -alkyle ou alcényle en C₁ à C₁₂. |
| R₇ | est -alkyle ou alcényle en C₁ à C₁₂. |

3. Liquide ionique selon la revendication 1 ou la revendication 2, **caractérisé en ce que** R₁, R₂, R₃, R₄, et R₅ sont H.

4. liquide ionique selon l'une quelconque des revendications 1 à 3, **caractérisé en ce que** R₆ est un butyle ou allyle et R₇ est un méthyle.

5. Mélange comprenant un liquide ionique selon l'une des revendications 1 à 4 et un activateur d'absorption.

6. Mélange selon la revendication 5, **caractérisé en ce que** l'activateur d'absorption est un liquide ionique ou un sel apparenté au liquide ionique.

7. Mélange selon la revendication 6, **caractérisé en ce que** le liquide ionique ou le sel apparenté au liquide ionique supplémentaire est de formule [Q⁺]_{b}[B^{b-}], dans laquelle [Q⁺] est choisi dans le groupe du 2-hydroxyéthanesulfonate, du p-xylène-2-sulfonate, ou d'une combinaison de ceux-ci.

8. Procédé pour la synthèse d'un liquide ionique selon l'une des revendications 2 à 4, par traitement d'un composé [C⁺][Y⁻] avec un composé [Z^{z+}][A⁻]_{z}.

9. Procédé selon la revendication 8, dans lequel
(a) [Y⁻] est choisi dans le groupe de BF₄⁻, Cl⁻, Br⁻, I⁻, ou OH⁻ et
(b) [Z^{z+}] est choisi dans le groupe de H⁺, un ion de métal alcalin, un ion de métal alcalino-terreux.

10. Dispositif pour l'absorption de SO₂, comprenant un réservoir, une entrée et une sortie pour des gaz de combustion, dans lequel le réservoir comprend un liquide ionique selon l'une des revendications 1 à 4 ou un mélange selon l'une des revendications 5 à 7.

11. Dispositif selon la revendication 10, **caractérisé par** un dispositif de nébulisation, de formation de film ou de formation de mousse.

12. Dispositif selon la revendication 10 ou la revendication 11, **caractérisé en ce qu'**il comprend une zone d'absorption dans laquelle le liquide ionique peut être exposé à du SO₂ et une zone de désorption où le SO₂ peut être désorbé, dans lequel un dispositif de chauffage est présent dans la zone de désorption.

13. Utilisation d'un liquide ionique comprenant un composé de formule [C⁺] [A⁻], dans laquelle
[A⁻] est 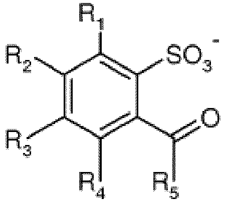 et
| | |
|---|---|
| R₁, R₂, R₃, R₄ | sont indépendamment -H ; -alkyle ou alcényle en C₁ à C₁₂ ; -OH ; -SH ; -CN ; -F ; -Cl ; -Br ; -I ; |
| R₅ | est -H, -alkyle ou alcényle en C₁ à C₁₂. |

14. Utilisation selon la revendication 13, **caractérisée en ce que** le composé de [C⁺] [A⁻] est un composé selon l'une des revendications 1 à 4 ou un mélange selon l'une des revendications 5 à 7 pour l'absorption de SO₂.
